# EUROPEAN PATENT SPECIFICATION

(11) **EP 1 769 804 B1**
(45) Date of publication and mention of the grant of the patent: **19.09.2012**
(21) Application number: 06027055.0
(22) Date of filing: 19.08.1999
(51) Int. Cl.: A61K 38/29, A61P 19/08, A61K 33/06, A61K 31/59

(54) **hPTH(1-34) for use in preventing or reducing the incidence or severity of vertebral and / or non vertebral fracture in a male human**
hPTH(1-34) zur Verwendung in der Vorbeugung und Reduzierung des Auftretens oder der Schwere von Knochenbrüchen bei Männern
hPTH(1-34) pour son utilisation dans la prévention ou la sévérité de fractures osseuses chez le mâle humain

(30) Priority: 19.08.1998 US 97151 P; 10.09.1998 US 99746 P
(43) Date of publication of application: 04.04.2007
(62) Divisional of application: 02079227.1
(73) Proprietor: ELI LILLY AND COMPANY, Indianapolis, IN 46285 (US)
(72) Inventor: Hock, Janet M., Indianapolis, IN 46250 (US); Gaich, Gregory A., Indianapolis, IN 46220 (US); Dere, Willard, H., Ascot, Berkshire, SL59JQ (GB)
(74) Representative: Fisher, Adrian John

(56) References cited:
- WO-A1-98/14478
- WO-A2-99/12561
- DATABASE WPI Section Ch, Week 199706 Thomson Scientific, London, GB; Class B04, AN 1997-061733 XP002127206 & JP 08 310965 A (CHUGAI PHARM CO LTD) 26 November 1996 (1996-11-26)
- EJERSTED C ET AL: "HUMAN PARATHYROID HORMONE (1-34) AND (1-84) INCREASE THE MECHANICAL STRENGTH AND THICKNESS OF CORTICAL BONE IN RATS" JOURNAL OF BONE AND MINERAL RESEARCH, vol. 8, no. 9, September 1993 (1993-09), pages 1097-1101, XP000857986 NEW YORK, NY, US ISSN: 0884-0431
- WHITFIELD J F ET AL: "Comparison of the ability of recombinant human parathyroid hormone, rhPTH-(1-84), and hPTH-(1-31)NH2 to stimulate femoral trabecular bone growth in ovariectomized rats." CALCIFIED TISSUE INTERNATIONAL, vol. 60, no. 1, January 1997 (1997-01), pages 26-29, XP002249487 UNITED STATES ISSN: 0171-967X
- JEROME C P ET AL: "EFFECT OF TREATMENT FOR 3 MONTHS WITH HUMAN PARATHYROID HORMONE 1-34 PEPTIDE IN OVARIECTOMIZED CYNOMOLGUS MONKEYS. (MACACA FASCICULARIS)" BONE, vol. 17, no. 4, October 1995 (1995-10), pages 415S-420S, XP000857980 PERGAMON PRESS. OXFORD, GB ISSN: 8756-3282
- PATENT ABSTRACTS OF JAPAN vol. 1996, no. 07, 31 July 1996 (1996-07-31) & JP 08 073376 A (ASAHI CHEM IND CO LTD), 19 March 1996 (1996-03-19)
- LINSAY R ET AL: "RANDOMISED CONTROLLED STUDY OF EFFECT OF PARATHYROID HORMONE ON VERTEBRAL-BONE MASS AND FRACTURE INCIDENCE AMONG POSTMENOPAUSAL WOMEN ON OESTROGEN WITH OSTEOPOROSIS" LANCET THE, vol. 350, 23 August 1997 (1997-08-23), pages 550-555, XP002127205 LANCET LIMITED. LONDON, GB ISSN: 0140-6736
- HIRANO T ET AL: "ANABOLIC EFFECTS OF HUMAN BIOSYNTHETIC PARATHYROID HORMONE FRAGMENT (1-34), LY333334, ON REMODELING AND MECHANICAL PROPERTIES OF CORTICAL BONE IN RABBITS" JOURNAL OF BONE AND MINERAL RESEARCH, vol. 14, no. 4, April 1999 (1999-04), pages 536-545, XP000857985 NEW YORK, NY, US ISSN: 0884-0431
- MITLAK BRUCE H ET AL: "Sequential effects of chronic human PTH (1-84) treatment of estrogen-deficiency osteopenia in the rat." JOURNAL OF BONE AND MINERAL RESEARCH, vol. 11, no. 4, 1996, pages 430-439, XP009014793 ISSN: 0884-0431
- OXLUND H ET AL: "Parathyroid hormone (1-34) and (1-84) stimulate cortical bone formation both from periosteum and endosteum." CALCIFIED TISSUE INTERNATIONAL, vol. 53, no. 6, 1993, pages 394-399, XP009014823 ISSN: 0171-967X
- R.M. NEER ET AL.: "The use of parathyroid hormone plus 1,25-dihydroxyvitamin D to increase trabecular bone in osteoporotic men and postmenopausal women" OSTEOPOROSIS, vol. 2, 1987, pages 829-835, XP002549387
- FUJITA T: "PARATHYROID HORMONE IN THE TREATMENT OF OSTEOPOROSIS" BIODRUGS: CLINICAL IMMUNOTHERAPEUTICS, vol. 15, no. 11, 1 January 2001 (2001-01-01), pages 721-728, XP008012814 BIOPHARMACEUTICALS AND GENE THERAPY, ADIS INTERNATIONAL, FR ISSN: 1173-8804

## Description

### TECHNICAL FIELD

This invention relates to methods for reducing the likelihood and/or severity of bone fracture by administering a parathyroid hormone. More particularly, the invention relates to a method of reducing the incidence of vertebral fracture, reducing the incidence of multiple vertebral fractures, reducing the severity of vertebral fracture, and/or reducing the incidence of non-vertebral fracture.

### BACKGROUND OF THE INVENTION

Existing agents such as estrogen, bisphosphonates, fluoride, or calcitonin can prevent bone loss and induce a 3 - 5% increase of bone mass by refilling the remodeling space, but net bone formation is not significantly stimulated. The retention of bone by inhibition of bone turnover may not be sufficient protection against fracture risk for patients who already have significant bone loss. Anabolic agents that increase bone strength by stimulating bone formation preferentially may provide better protection against fracture in patients with established osteoporosis.

Parathyroid hormone (PTH) is a secreted, 84 amino acid product of the mammalian parathyroid gland that controls serum calcium levels through its action on various tissues, including bone. The N-terminal 34 amino acids of bovine and human PTH (PTH(1-34)) is deemed biologically equivalent to the full length hormone. Other amino terminal fragments of PTH (including 1-31 and 1-38 for example), or PTHrP (PTH-related peptide/protein) or analogues of either or both, that activate the PTH/PTHrP receptor (PTH1 receptor) have shown similar biologic effects on bone mass, although the magnitude of such effects may vary.

Studies in humans with various forms of PTH have demonstrated an anabolic effect on bone, and have prompted significant interest in its use for the treatment of osteoporosis and related bone disorders. The significant anabolic effects of PTH on bone, including stimulation of bone formation which results in a net gain in bone mass and/or strength, have been demonstrated in many animal models and in humans.

It is commonly believed that PTH administration in humans and in relevant animal models has a negative effect on cortical bone. In fact, naturally occurring increases in endogenous PTH, which occur in the disorder hyperparathyroidism, result in thinning of cortical bone accompanied by an increase in connectivity and mass of trabecular bone. Past studies suggest that when Haversian cortical bone (found in humans and higher mammals) remodels under the influence of PTH, there will be a re-distribution of bone such that cortical bone mass and strength decrease, while trabecular bone increases in mass and strength. For example, in published clinical studies of administering PTH, cortical bone mass decreased after treatment with exogenous PTH and these findings have raised concern that the treatment of PTH will lead to reduced cortical bone mass and strength. One concern raised by such studies is that there would be a loss of total skeletal bone mass due to the loss of cortical bone. This is of high clinical relevance as, in osteoporosis, the greater loss of trabecular bone compared to loss of cortical bone, means that mechanical loading is predominantly borne by the remaining cortical bone. Continued loss of cortical bone would increase the fracture risk. Therefore, it is important that a therapeutic agent for osteoporosis maintain or increase a subjects residual cortical bone.

The effects of PTH on cortical bone have been investigated in nonhuman animals with Haversian remodeling, such as dogs, ferrets, sheep and monkeys, but sample sizes are typically too small for reliable statistical analysis. The impact of the changes induced by PTH treatment on mechanical properties of cortical bone in such animals remains unknown. Published studies of rodents have shown increased cortical bone mass during administration of PTH but a loss of this benefit after withdrawal of PTH. However, rodent cortical bone has a distinctly different structure from Haversian cortical bone, and remodels by surface appositional formation and resorption, rather than by intracortical remodeling of osteons. Furthermore, technological limitations in biomechanical testing on the relatively short bones of rodents give rise to artifacts of measurement when an agent, such as a PTH, alters bone geometry to thicken the bone. Such artifacts make extrapolation of rat cortical bone responses to those of humans or other animals with osteonal remodeling unreliable. Therefore, the existing data for animals, like humans, undergoing Haversian remodeling indicates that PTH may have an adverse impact on cortical bone, causing net loss of bone mass through depletion of cortical bone.

As a consequence, it has been a popular belief regarding the action of PTH that patients require concurrent or subsequent treatment with an antiresorptive to minimize loss of bone induced by PTH. In fact, this model has been the basis for several clinical studies in women. For example, three clinical studies have used PTH in post-menopausal women on concurrent therapy with calcitonin or estrogen, or in premenopausal women taking GnRH agonist, Synarel, for endometriosis. The opposing effects of estrogen and PTH on cortical bone turnover make it particularly difficult to observe effects of just PTH during combination therapy with these two agents.

There remains a need for a method for employing a PTH to increase strength and stiffness of bone in humans and other animals exhibiting Haversian remodeling, and for reducing the incidence of fracture of bones in these animals. Furthermore, there remains a need for a method for increasing the quality and amount of cortical bone.

### SUMMARY OF THE INVENTION

The present invention provides

Human PTH(1-34) for use in preventing or reducing the incidence or seventy of vertebral and/or non-vertebral fracture in a male human at risk for or having osteoporosis, wherein the PTH(1-34) is administered by subcutaneous injection at a dose of 10 to 40 µg/day.

### BRIEF DESCRIPTION OF THE DRAWINGS

Figure 1 illustrates the percent change in DXA measures of whole bone mineral content in control and treatment groups.
Figures 2A-C illustrate the percent change in DXA measures of the spine for control and treatment groups in the lumbar vertebrae 2-4 for bone area (A), bone mineral content (B), and bone mineral density (C).
Figures 3A and 3B illustrate the increase in bone mass (A) and bone strength (B) in lumbar vertebrae of primates treated with a parathyroid hormone.
Figures 4A and 4B illustrate the increase in strength of femur neck (A) and the constant strength of humerus mid-diaphysis (B) in primates treated with a parathyroid hormone.
Figure 5 illustrates activation of bone formation rates on endosteal and periosteal surfaces of the midshaft humerus.
Figure 6 illustrates the histogram analysis of the shift in bone voxel densities in lumbar vertebra, resulting from PTH treatment compared to control. Note the increase in density in cortical bone compartment after withdrawal of PTH treatment.
Figure 7 illustrates increases in lumbar spine BMD through 23 months of treatment of patients with either 20 µg//day PTH or 40 µg//day PTH, compared to placebo treated controls.
Figure 8 illustrates increases in femur and hip neck BMD through 24 months of treatment of patients with either 20 µg//day PTH or 40 µg//day PTH, compared to placebo treated controls.

### DETAILED DESCRIPTION

The invention can be used for increasing bone toughness and/or stiffness, and/or reducing incidence of fracture in a subject by administering a parathyroid hormone. It can be employed to increase stiffness and/or toughness at a site of a potential trauma or at a site of an actual trauma. Trauma generally includes fracture, surgical trauma, joint replacement, orthopedic procedures, and the like. Increasing bone toughness and/or stiffness generally includes increasing mineral density of cortical bone, increasing strength of bone, increasing resistance to loading, and the like. Reducing incidence of fracture generally includes reducing the likelihood or actual incidence of fracture for a subject compared to an untreated control population.

As used herein, ultimate force refers to maximum force that a bone specimen sustains; stiffness refers to the slope of the linear portion of a load-deformation curve; and work to failure refers to the area under the load-deformation curve before failure. Each of these can be measured and calculated by methods standard in the bone study art. These parameters are structural properties that depend on intrinsic material properties and geometry, and can be determined as described in Turner CH, Burr DB 1993 "Basic biomechanical measurements of bone: a tutorial." Bone 14:595-608, which is incorporated herein by reference. Ultimate force, stiffness, and work to failure can be normalized to obtain intrinsic material properties such as ultimate stress, elastic modulus, and toughness, which are independent of size and shape. As used herein, ultimate stress refers to maximum stress that a specimen can sustain; elastic modulus refers to material intrinsic stiffness; and toughness refers to resistance to fracture per unit volume. Each of these can be determined by methods known in the art. Id. Femoral bone strength, as referred to herein, can be measured at the femur neck, or at the midshaft typically using three-point or four-point bending at the latter site.

### Bone Trauma

The invention is of benefit to a subject (a male human) that may suffer or have suffered trauma to one or more bones.

Risk factors for osteoporosis are known in the art and include hypogonadal conditions in men, irrespective of age, conditions, diseases or drugs that induce hypogonadism, nutritional factors associated with osteoporosis (low calcium or vitamin D being the most common), smoking, alcohol, drugs associated with bone loss (such as glucocorticoids, thyroxine, heparin, lithium, anticonvulsants etc.), loss of eyesight that predisposes to falls, space travel, immobilization, chronic hospitalization or bed rest, and other systemic diseases that have been linked to increased risk of osteoporosis. Indications of the presence of osteoporosis are known in the art and include radiological evidence of at least one vertebral compression fracture, low bone mass (typically at least 1 standard deviation below mean young normal values), and/or atraumatic fractures.

Osteoporosis can lead, for example, to vertebral and/or non-vertebral fractures. Examples of non-vertebral fractures include a hip fracture, a fracture of a distal forearm, a fracture of a proximal humerus, a fracture of a wrist, a fracture of a radius, a fracture of an ankle, a fracture of an humerus, a fracture of a rib, a fracture of a foot, a fracture of a pelvis, or a combination of these. The invention can be used to decrease the risk of such fractures or for treating such fractures. The risk of fracture is diminished and the healing of a fracture is aided by increasing the strength and/or stiffness of bone, for example, in the hip, the spine or both. The invention can benefit a subject at any stage of osteoporosis, but especially in the early and advanced stages.

The present invention is effective to prevent or reduce the incidence of fractures in a subject with or at risk of progressing to osteoporosis. For example, the present invention can reduce the incidence of vertebral and/or non-vertebral fracture, reduce the severity of vertebral fracture, reduce the incidence of multiple vertebral fracture, improve bone quality, and the like. In another embodiment, the invention can benefit patients with low bone mass or prior fracture who are at risk for future multiple skeletal fractures, such as patients in which spinal osteoporosis may be progressing rapidly.

Other subjects can also be at risk of or suffer bone trauma and can benefit from the method of the invention. For example, a wide variety of subjects at risk of one or more of the fractures identified above, can anticipate surgery resulting in bone trauma, or may undergo an orthopedic procedure that manipulates a bone at a skeletal site of abnormally low bone mass or poor bone structure, or deficient in mineral. For example, recovery of function after a surgery such as a joint replacement (e.g. knee or hip) or spine bracing, or other procedures that immobilize a bone or skeleton can improve due to the method of the invention. The the invention can also aid recovery from orthopedic procedures that manipulate a bone at a site of abnormally low bone mass or poor bone structure, which procedures include surgical division of bone, including osteotomies, joint replacement where loss of bone structure requires restructuring with acetabulum shelf creation and prevention of prosthesis drift, for example. Other suitable subjects for practice of the present invention include those suffering from hypoparathyroidism or kyphosis, who can undergo trauma related to, or caused by, hypoparathyroidism or progression of kyphosis.

### Bone Toughness and Stiffness

The invention reduces the risk of trauma or aids recovery from trauma by increasing bone toughness, stiffness or both. Generally toughness or stiffness of bone results from mass and strength of cortical, trabecular, and cancellous bone. The invention can provide levels of bone toughness, stiffness, mass, and/or strength within or above the range of the normal population. Preferably the invention provides increased levels relative to the levels resulting from trauma or giving rise to risk of trauma. Increasing toughness, stiffness, or both decreases risk or probability of fracture compared to an untreated control population.

Certain characteristics of bone when increased provide increased bone toughness and/or stiffness. Such characteristics include bone mineral density (BMD), bone mineral content (BMC), activation frequency or bone formation rate, trabecular number, trabecular thickness, trabecular and other connectivity, periosteal and endocortical bone formation, cortical porosity, cross sectional bone area and bone mass, resistance to loading, and/or work to failure. An increase in one or more of these characteristics is a preferred outcome of the invention.

Certain characteristics of bone, such as marrow space and elastic modulus when decreased provide increased toughness and/or stiffness of bone. Younger (tougher and stiffer) bone has crystallites that are generally smaller than crystallites of older bone. Thus, generally reducing the size of bone crystallites increases toughness and stiffness of bone, and can reduce incidence of fracture. In addition, maturing the crystallites of a bone can provide additional desirable characteristics to the bone, including increased toughness and stiffness of bone and/or can reduced incidence of fracture. A decrease in one or more of these characteristics can be a preferred outcome of the invention.

The invention is effective for increasing the toughness and/or stiffness of any of several bones. For example, the invention can increase the toughness and/or stiffness of bones including a hip bone, such as an ilium, a leg bone, such as a femur, a bone from the spine, such as a vertebra, or a bone from an arm, such as a distal forearm bone or a proximal humerus. This increase in toughness and/or stiffness can be found throughout the bone, or localized to certain portions of the bone. For example, toughness and/or stiffness of a femur can be increased by increasing the toughness and/or stiffness of a femur neck or a femur trochantera. Toughness and/or stiffness of a hip can be increased by increasing the toughness and/or stiffness of an iliac crest or iliac spine. Toughness and/or stiffness of a vertebra can be increased by increasing the toughness and/or stiffness of a pedicle, lamina, or body. Advantageously, the effect is on vertebra in certain portions of the spine, such as cervical, thoracic, lumbar, sacral, and/or coccygeal vertebrae. Preferably the effect is on one or more mid-thoracic and/or upper lumbar vertebrae.

The increase in toughness and/or stiffness can be found in each of the types of bone, or predominantly in one type of the bone. Types of bone include spongy (cancellous, trabecular, or lamellar) bone and compact (cortical or dense) bone and the fracture callus. The invention preferably increases toughness and/or stiffness through its effects on cancellous and cortical bone, or on cortical bone alone. Trabecular bone, bone to which connective tissue is attached can also be toughened and/or stiffened by the invention. For example, it is advantageous to provide additional toughness at a site of attachment for a ligament, a tendon, and/or a muscle.

In another aspect of the invention, increasing toughness or stiffness can reduce incidence of fracture. In this aspect, increasing toughness or stiffness can include reducing incidence of vertebral fracture, reducing incidence of severe fracture, reducing incidence of moderate fracture, reducing incidence of non-vertebral fracture, reducing incidence of multiple fracture, or a combination thereof.

### Parathyroid Hormone

The invention uses human PTH(1-34), also known as teriparatide. Stabilized solutions of human PTH(1-34), such as recombinant human PTH(1-34) (rhPTH(1-34), that can be employed in the present method are described in WO99/29337 Crystalline forms of human PTH(1-34) that can be employed in the present method are described in WO 99/31137

### Administering Parathyroid Hormone

A parathyroid hormone can typically be administered parenterally, preferably by subcutaneous injection, by methods and in formulations well known in the art. Stabilized formulations of human PTH(1-34) that can advantageously be employed in the present method are described in WO99/29337 . This patent application also describes numerous other formulations for storage and administration of parathyroid hormone. A stabilized solution of a parathyroid hormone can include a stabilizing agent, a buffering agent, a preservative, and the like.

The stabilizing agent incorporated into the solution or composition includes a polyol which includes a saccharide, preferably a monosaccharide or disaccharide, e.g., glucose, trehalose, raffinose, or sucrose; a sugar alcohol such as, for example, mannitol, sorbitol or inositol, and a polyhydric alcohol such as glycerine or propylene glycol or mixtures thereof. A preferred polyol is mannitol or propylene glycol. The concentration of polyol may range from about 1 to about 20 wt-%, preferably about 3 to 10 wt-% of the total solution.

The buffering agent employed in the solution or composition may be any acid or salt combination which is pharmaceutically acceptable and capable of maintaining the aqueous solution at a pH range of 3 to 7, preferably 3-6. Useful buffering systems are, for example, acetate, tartrate or citrate sources. Preferred buffer systems are acetate or tartrate sources, most preferred is an acetate source. The concentration of buffer may be in the range of about 2 mM to about 500 mM, preferably about 2 mM to 100 mM.

The stabilized solution or composition may also include a parenterally acceptable preservative. Such preservatives include, for example, cresols, benzyl alcohol, phenol, benzalkonium chloride, benzethonium chloride, chlorobutanol, phenylethyl alcohol, methyl paraben, propyl paraben, thimerosal and phenylmercuric nitrate and acetate. A preferred preservative is m-cresol or benzyl alcohol; most preferred is m-cresol. The amount of preservative employed may range from about 0.1 to about 2 wt-%, preferably about 0.3 to about 1.0 wt-% of the total solution.

Thus, the stabilized teriparatide solution can contain mannitol, acetate and m-cresol with a predicted shelf-life of over 15 months at 5°C.

The parathyroid hormone compositions can, if desired, be provided in a powder form containing not more than 2% water by weight, that results from the freeze-drying of a sterile, aqueous hormone solution prepared by mixing the selected parathyroid hormone, a buffering agent and a stabilizing agent as above described. Especially useful as a buffering agent when preparing lyophilized powders is a tartrate source. Particularly useful stabilizing agents include glycine, sucrose, trehalose and raffinose.

In addition, parathyroid hormone can be formulated with typical buffers and excipients employed in the art to stabilize and solubilize proteins for parenteral administration. Art recognized pharmaceutical carriers and their formulations are described in Martin, "Remington's Pharmaceutical Sciences," 15th Ed.; Mack Publishing Co., Easton (1975). A parathyroid hormone can also be delivered via the lungs, mouth, nose, by suppository, or by oral formulations.

The parathyroid hormone is formulated for administering a dose effective for increasing toughness and/or stiffness of one or more of a subject's bones and/or for reducing the likelihood and/or severity of bone fracture. Preferably, an effective dose provides an improvement in cortical bone structure, mass, and/or strength. Preferably, an effective dose reduces the incidence of vertebral fracture, reduces the incidence of multiple vertebral fractures, reduces the severity of vertebral fracture, and/or reduces the incidence of non-vertebral fracture. Preferably, a subject receiving parathyroid hormone also receives effective doses of calcium and vitamin D, which can enhance the effects of the hormone. An effective dose of parathyroid hormone is 10 to 40 µg/day.

The hormone can be administered regularly (e.g., once or more each day or week), intermittently (e.g., irregularly during a day or week), or cyclically (e.g., regularly for a period of days or weeks followed by a period without administration). Preferably PTH is administered once daily for 1-7 days for a period ranging from 3 months for up to 3 years in osteoporotic patients. Preferably, cyclic administration includes administering a parathyroid hormone for at least 2 remodeling cycles and withdrawing parathyroid hormone for at least 1 remodeling cycle. Another preferred regime of cyclic administration includes administering the parathyroid hormone for at least about 12 to about 24 months and withdrawing parathyroid hormone for at least 6 months. Typically, the benefits of administration of a parathyroid hormone persist after a period of administration. The benefits of several months of administration can persist for as much as a year or two, or more, without additional administration.

### Uses of Formulations of a Parathryoid Hormone

kit can be used with the present invention. The kit can contain a vial which contains a formulation of the present invention and suitable carriers, either dried or in liquid form. The kit further includes instructions in the form of a label on the vial and/or in the form of an insert included in a box in which the vial is packaged, for the use and administration of the compounds. The instructions can also be printed on the box in which the vial is packaged. The instructions contain information such as sufficient dosage and administration information so as to allow a worker in the field to administer the drug. It is anticipated that a worker in the field encompasses any doctor, nurse, or technician who might administer the drug.

### EXAMPLES

### Example 1 - Increased Bone Strength and Density Upon

### Administration of rhPTH(1-34) to Monkeys (for reference)

### Experimental Procedures

### General

The live phase of the study used feral, adult (closed growth plates) cynomolgus primates (*Macaca fascicularis*), weighing 2.77±0.03 kg (mean ± standard error of the mean [SEM]). Monkeys were held in quarantine for 3 months, then started on a diet containing 0.3% calcium, 0.3% phosphate, and 250 IU vitamin D3/100 g diet, and given fluoridated water (1 ppm fluoride) ad libitum. The calcium content corresponded to 1734 mg calcium/2000 calories. After 1 month on the diet, animals were sorted into groups of 21 or 22, sham operated or ovariectomized. Once daily subcutaneous injections of vehicle (sham and ovariectomized controls) or rhPTH(1-34), at 1 µg/kg (PTH1) or 5 µg/kg (PTH5), were started 24 hours after ovariectomy. Animals were treated for either 18 months (PTH1 and PTHS), or for 12 months followed by withdrawal of treatment (PTH1-W and PTH5-W).

The study groups were divided as shown in Table 1.

**Table 1 - - Study Groups for Primate Study**

| Group | Abbreviation | Monkeys at Outset (n=128) | Monkeys in Final Analyses (n=121) |
|---|---|---|---|
| Sham ovariectomized, 18 months vehicle | Sham | 21 | 21 |
| Ovariectomized, 18 months vehicle | OVX | 22 | 20 |
| Ovariectomized, 18 months 1 µg rhPTH(1-34)/kg/day | PTH1 | 21 | 19 |
| Ovariectomized, 12 months 1 µg rhPTH(1-34/kg/day,6 months vehicle | PTH1-W | 21 | 20 |
| Ovariectomized, 18 months 5µg rhPTH(1-34)/kg/day | PTH5 | 22 | 21 |
| Ovariectomized, 12 months 5µg rhPTH(1-34)/kg/day, 6 months vehicle | PTH5-W | 21 | 20 |

Serum and urinary samples were taken 24 hours after vehicle or rhPTH(1-34) injection at 3-month intervals. A sparse sampling design of 5 monkeys in each rhPTH(1-34)-treated group was used for pharmacokinetics, with sampling (spanning 0 to 240 minutes each time) at baseline, 7, 11, and 17 months. At 0 time and at 6-month intervals, total skeleton and spine (L-2 to L-4) bone mass were assessed by dual-energy x-ray absorptiometry (DXA); peripheral quantitative computed x-ray tomography (pQCT) was used to assess bone mass in the midshaft and distal radii, and the proximal tibia. Iliac biopsies were taken at 6 and 15 months for histomorphometry. All animals were euthanized after 18 months.

Biomechanical tests were done on lumbar vertebrae L-3 to L-4, the femur neck, humerus midshaft, and on a cortical bone specimen machined from the femur diaphysis (measures defined in Table 2). Conventional static and dynamic histomorphometry were done (measures described in Table 6) on the humerus midshaft, lumbar vertebra L-2, femoral neck, femur midshaft, the radius midshaft and distal radius. Initial statistical analyses compared all groups to vehicle-treated ovariectomized controls. The data is suitable for additional exploratory analyses to examine dose dependency, effects of withdrawal, interactions between outcomes, and changes in time by methods known to those of skill in the art. All assays were conducted and determined by methods known in the art.

For certain experimental subjects, cortical bone of the humerus was examined by histomorphometry and by polarized Fourier transform infrared microscopy. The Fourier transform infrared microscopy was conducted by an adaptation of known methods for such microscopy.

### 3D Finite Element Modeling Studies

These studies determined 3D finite element modeling data on vertebra from monkeys of the study dosed with PTH for 18 months. Excised L-5 vertebra in 50% ethanol/saline from the ovariectomized (n=7) and PTH (n=7) groups were serially scanned in 500 µm steps by quantitative computed tomography (QCT, Norland, Ft. Atkinson, WI), using 70x70 µm pixels. Each of the 500 µm cross-sections was analyzed for volumetric bone mineral density (BMD, mg/cc), bone mineral content (BMC, mg), cross-sectional area (X-Area), cancellous bone volume (BV/TV), trabecular thickness (Tb.Th), and connectivity (node density, strut analysis). Pixels in each serial scan were averaged to create 490x490x500 µm voxels. The serial scans were then stacked and a triangular surface mesh generated for each bone using the "marching cubes" algorithm (see e.g. Lorensen and Cline 1987 "Marching cubes, a high resolution 3D surface construction algorithm." Computer Graphics 21, 163-169). A smoothed version of each surface mesh was then used to generate a tetrahedral mesh for 3D finite-element modeling.

Young's modulus for each tetrahedral element was derived from the original voxel densities and material properties from a beam of cortical bone milled from the femoral diaphysis of the monkeys. Each tetrahedral mesh was rotated so that the bottom surface of each vertebra was aligned with a plane. Linear elastic stress analysis was then performed for each L-5 model in which a distributed load of 100 N was applied to the top surface of the centrum, perpendicular to the bottom plane while the bottom surface was fixed in the direction of loading. The resulting axial strain contours were evaluated, as were the BMD distributions, and compared between PTH and ovariectomized. At this resolution, the density of each voxel is dependent on the extent to which each voxel is filled with bone as opposed to soft tissue.

### Results

Reports of differences in the text are statistically significant, p<0.05. All animals gained 4% to 9% of initial body weight during the study independent of treatment.

### Serum and Urine Measures

Serum estradiol levels at 3 and 18 months were below 5 pg/mL in all ovariectomized monkeys. When measures of calcium homeostatis were compared to sham controls, ovariectomized controls had lower serum calcium and phosphate and 1,25-dihydroxyvitamin D levels, but did not differ in endogenous PTH, urinary cyclic adenosine monophosphate (cAMP), urinary calcium, urinary creatinine, or serum urea nitrogen measured 24 hours after last injection. Animals treated with rhPTH(1-34) had lower serum phosphate, lower endogenous PTH, and higher 1,25-dihydroxyvitamin D and urinary cAMP compared to ovariectomized. Serum bone formation marker assays showed that ovariectomized monkeys had low serum total alkaline phosphatase (ALP) and osteocalcin compared to shams, and rhPTH(1-34) restored levels back to sham values. Urinary C-telopeptide (CrossLaps) excretion, used as a biochemical marker of bone resorption, was not altered by rhPTH(1-34) compared to ovariectomized controls.

### Bone Mass

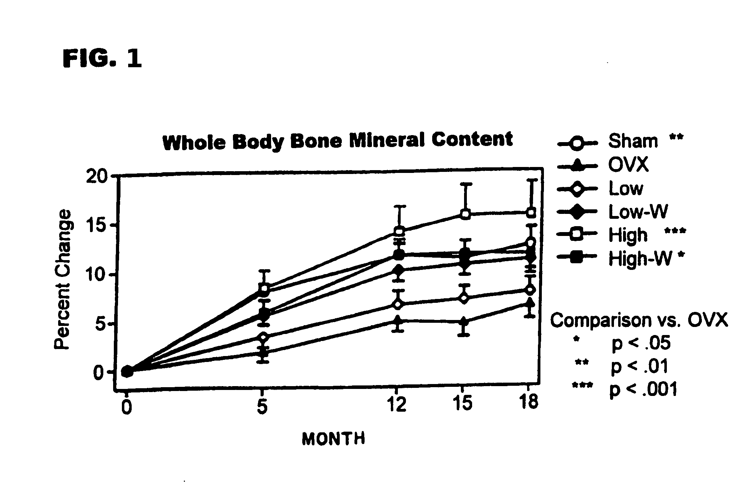

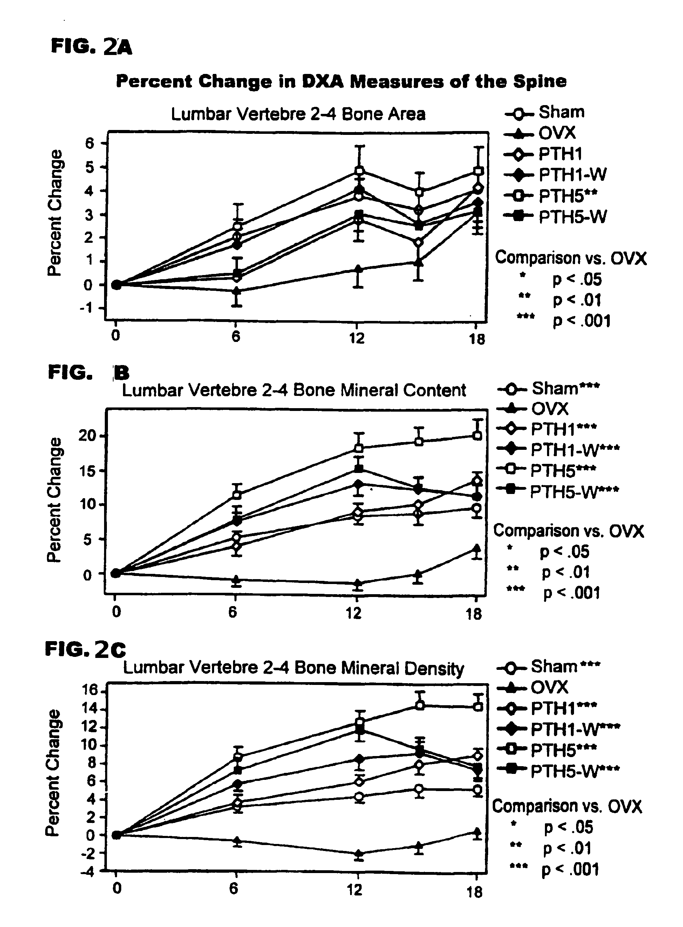

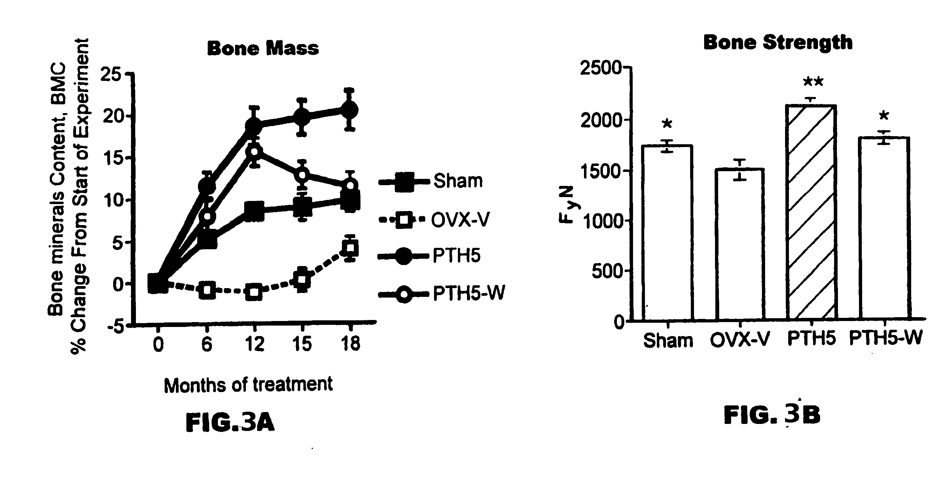

Overall skeletal bone mass, expressed as total body BMC, was increased significantly by PTH(1-34) (Fig. 1). Spine bone mineral density (BMD) remained stable in ovariectomized controls for 18 months, while sham controls gained 3 approximately 5% above baseline (Figs. 2-2C and 3A). rhPTH(1-34) increased spine BMD by 7% to 14% and whole body bone mineral content (BMC, Figure 3) by up to 6% compared to baseline (Figs. 2A-2C and 3A). Spine bone mineral content also increased (Fig. 3A). In rhPTH(1-34)-treated primates, the magnitude of these increases was significantly higher than that of ovariectomized controls, and matched (PTH1) or exceeded (PTH5) that of shams. rhPTH(1-34) did not alter BMD of the midshaft or distal radius. The cross-sectional area of the midshaft increased by 7% in the PTH5 group. In the proximal tibia, there was no increase in cross-sectional area but rhPTH(1-34) increased BMC and BMD compared to ovariectomized controls. Six months after treatment was withdrawn, BMD and BMC in the spine and femur neck remained higher than ovariectomized controls, with no change in the cortical midshaft of the humerus.

### Bone Strength

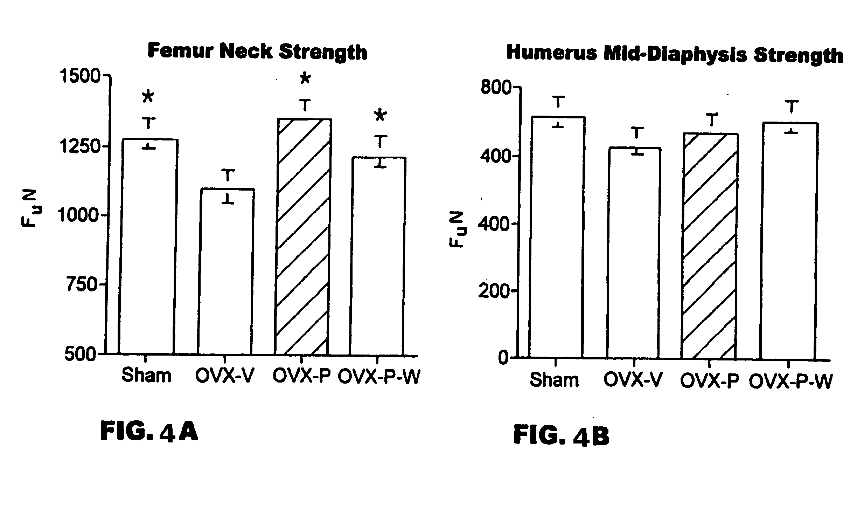

rhPTH(1-34) increased strength (F_{y}) in the vertebrae by up to 43% (Table 2 and 3, Fig. 3B). rhPTH(1-34) improved strength in the femur neck (fᵤ) by up to 12% (Tables 2 and 4, Fig. 4A), rhPTH(1-34) did not alter measures in the cortical diaphysis of the humerus midshaft (Tables 2 and 5), or the material properties of beam specimens machined from the femoral diaphysis (Tables 2 and 4, Fig. 4B) when compared to ovariectomized controls. In animals treated with rhPTH(1-34) for 12 months and then withdrawn from treatment for 6 months, bone strength measures remained significantly higher than ovariectomized controls (Tables 2-5 Figs. 3B and 4A).

**Table 2 Variables Reported for the Third and Fourth Lumbar Vertebrae (L-3 and L-4), Humerus Midshaft, Proximal Femoral Neck, and Femoral Beam Specimens**

| Variable | | Units | Description |
|---|---|---|---|
| Lumbar Vertebrae, L-3 and L-4 | | | |
| | A | mm² | Cross-sectional area |
| | F. | N | Yield force is the force at a 0.2% offset |
| | S | N/mm | Slope of the linear portion of the force-displacement curve (stifrness) |
| | σγ | Mpa | Yield stress |
| | E | Mpa | Young's modulus |

| Humerus Midshaft | | | |
|---|---|---|---|
| | t | mm | Average cortical thickness |
| | Fᵤ | N | Ultimate force is the maximum force the specimen can withstand |
| | S | N/mm | Slope of the linear portion of the force-displacement curve (stiffness) here's stiffness |
| | mJ/U | N-mm | Area under the load-displacement curve (U-work to failure) |

| Proximal Femoral Neck | | | |
|---|---|---|---|
| | Fᵤ | N | Ultimate force is the maximum force the specimen can withstand |

| Femur Diaphysical Beam Specimens | | | |
|---|---|---|---|
| | σᵤ | Mpa | Ultimate stress |
| | E | Gpa | Young's modulus |
| | u | J/m³ | Toughness |
| | εᵤ | | Ultimate strain |

**Table 3 Biomechanical Measures of Strength in the Spine (Lumbar Vertebrae, L-3 and L-4 Combined) of Ovariectomized Primates at 18 Months**

| Variable (units)^{a} | Sham | OVX Control | PTH1 | PTH1-W | PTH5 | PTH5-W |
|---|---|---|---|---|---|---|
| A(mm²) | 90.3±2.1^{b} | 86.1±2.3 | 883±2.0 | 90.9±2.3 | 87.3±2.7 | 82.8±2.1 |
| F_{y}(N) | 1738±52 | 1499±94^{a} | 1915±105^{a} | 1899±73^{a} | 2113±77^{3.0} | 1792±39^{a} |
| S(N/mm) | 7312±319 | 5805±476^{a} | 7701 ± 474^{a} | 7401±432^{a} | 8012 ±367^{a} | 7074±314^{a} |
| σ_{y} (Mpsa) | 19.4±0.6 | 17.3±1.0 | 21.9±1.3^{a} | 21.1±0.8^{a} | 24.6±1.1^{3.0} | 21.9±0.9^{a} |
| E(Mpa) | 650±32 | 546±49 | 717±48^{a} | 659±42 | 759±36^{a} | 698±41^{a} |

| | | | | | | |
|---|---|---|---|---|---|---|
| Abbreviations: OVX=ovariectomized; PTH1=rhPTH(1-34) µg/kg for 18 months; PTH1-W= withdrawal for 6 months after treatment with rhPTH(1-34) 1 µg/kg for 12 months; PTH5=rhPTH(1-34) 5 µg/kg for 18 months; PTH5-W=withdrawal for 6 months after treatment with rhPTH(1-34) 5 µg/kg for 12 months. ^{a}See Table 4.1 for description of variables ^{b}Data expressed as mean ± standard error of the mean (SEM) per group. ^{o}Statistically significant compared to OVX controls (p<0.05). ^{s}Statistically significant compared to sham controls (p<0.05). | | | | | | |

**Table 4 Biomechanical Measures of Material Properties of Equivalent Size Beam Specimens from Femur Diaphysis, and Biomechanical Measure of Strength of Femur Neck of Ovariectomized Primates at 18 Months**

| Variable (units)^{a} | Sham | OVX Control | PTH1 | PTH1-W | PTH5 | PTH5-W |
|---|---|---|---|---|---|---|
| σₐ (Mpa) | 222 ± 5^{b} | 216 ± 5 | 222 ± 4 | 214 ± 6 | 206 ± 6 | 208 ± 6 |
| E(Gpa) | 17.2 ± 0.6 | 16.4 ± 0.4 | 17.1 ± 0.4 | 16.6 ± 0.6 | 15.4 ± 0.6^{a} | 15.3 ± 0.6^{a} |
| u (mJ/m³) | 5.9 ± 0.3 | 5.8 ± 0.4 | 6.1 ± 0.4 | 5.3 ± 0.4 | 5.4 ± 0.4 | 6.1 ± 0.4 |
| εₐ | 0.035 ± 0.001 | 0.035 ± 0.002 | 0.036 ± 0.002 | 0.034 ± 0.002 | 0.034 ± 0.002 | 0.038 ± 0.002 |
| Proximal femur neck | | | | | | |
| Fₐ | 1288 ± 41 | 1105 ± 53^{a} | 1235 ± 43^{a} | 1258 ± 52^{a} | 1362 ± 30^{a} | 1213 ± 42 |

| | | | | | | |
|---|---|---|---|---|---|---|
| Abbreviations: OVX=ovariectomized; PTH1=rhPTH(1-34) 1 µg/kg for 18 months; PTH1-W= withdrawal after treatment with rhPTH(1-34) 1 µg/kg for 12 months; PTH5=rhPTH(1-34) 5 µg/kg for 18 months; PTH5-W=withdrawal after treatment with rhPTH(1-34) 5 µg/kg for 12 months. ^{a}See Table 4.1 for description of variables ^{b}Data expressed as mean ± standard error of the mean (SEM) per group. ^{o}Statistically significant compared to OVX controls (p<0.05). ^{s}Statistically significant compared to sham controls (p<0.05). | | | | | | |

**Table 5 Biomechanical Measures of Cortical Bone of the Midshaft of the Humerus of Ovariectomized Primates at 18 Months**

| Variable (units)^{a} | Sham | OVX Control | PTH1 | PTH1-W | PTH5 | PTH5-W |
|---|---|---|---|---|---|---|
| t(mm) | 1.74 ± 0.04^{b} | 1.63 ± 0.03^{a} | 1.68 ± 0.03 | 1.66 ± 0.04 | 1.80 ± 0.04^{a} | 1.72 ± 0.05 |
| F_{y}(N) | 725 ± 26 | 636 ± 26 | 654 ± 23 | 689 ± 23 | 680 ± 15^{a} | 707 ± 24 |
| S(N/mm) | 601 ± 23 | 520 ± 26 | 544 ± 23 | 573 ± 20 | 548 ± 18 | 573 ± 24 |
| U(ml) | 1797 ± 85 | 1542 ± 92 | 1641 ± 137 | 1751 ± 84 | 1804 ± 99 | 1775 ± 113 |

| | | | | | | |
|---|---|---|---|---|---|---|
| Abbreviations: OVX=ovariectomized; PTH1=rhPTH(1-34) 1 µg/kg for 18 months; PTH1-W= withdrawal after treatment with rhPTH(1-34) 1 µg/kg for 12 months; PTH5=rhPTH(1-34) 5 µg/kg for 18 months; PTH5-W=withdrawal after treatment with rhPTH(1-34) 5 µg/kg for 12 months. ^{a}See Table 4.1 for description of variables ^{b}Data expressed as mean ± standard error of the mean (SEM). ^{o}Statistically significant compared to OVX controls (p<0.05). ^{s}Statistically significant compared to sham controls (p<0.05). | | | | | | |

### Bone Histomorphometry

Although turnover rates were greater in ovariectomized than sham controls, there was no significant loss of bone volume in the iliac crest. As the tetracycline label given at 6 months was not detectable in many animals, only static parameters were measured for this time point. Static and dynamic histomorphometry data at 15 months showed that treatment with rhPTH(1-34) increased cancellous bone area compared to ovariectomized, and increased bone formation without increasing resorption measures above those measured in ovariectomized controls. Bone formation rate was increased progressively by higher doses of rhPTH( 1-34). Although cancellous bone remained increased compared to ovariectomized controls after withdrawal of rhPTH(1-34) following 12 months of treatment, bone formation and resorption reverted to that seen in ovariectomized controls, and bone turnover remained higher than in sham controls. rhPTH(1-34) did not affect mineralization, activation frequency, or remodeling periods. There were no differences in individual bone multicellular unit (BMU)-based bone balance between resorption and formation. In summary, rhPTH(1-34) increased cancellous bone by selective stimulation of bone formation.

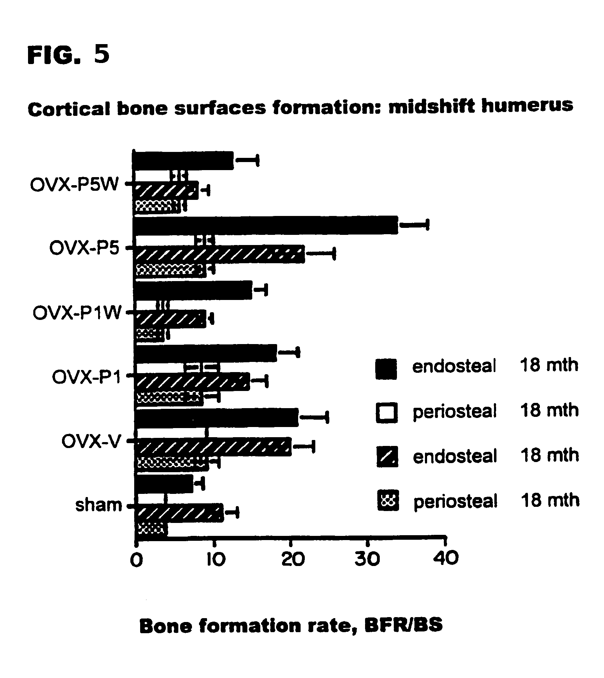

In the cortical bone of the humerus, where rhPTH(1-34) did not significantly modify BMD or bone strength measures, rhPTH(1-34) stimulated changes in the periosteal, endosteal, and intracortical compartments (Tables 6 and 17). Although there were no differences in total area or medullary area between groups, rhPTH(1-34) increased cortical area, and the PTH5 and PTH5-W groups had significantly more cortical bone, suggestive of increased cross-sectional moment of inertia, a measure of strength. The increase in area could be attributed to increased formation on both periosteal and endosteal surfaces (Fig. 5).

Sham controls and PTH5-W groups had reduced periosteal mineralizing surfaces compared to ovariectomized controls and the other rhPTH(1-34)-treated groups. Endocortical mineralizing surfaces were significantly greater in ovariectomized controls compared to shams and rhPTH(1-34) did not increase above ovariectomized control values. In intracortical remodeling, there were more resorption spaces in ovariectomized animals, and activation frequency was greater in ovariectomized, PTH1, and PTH5 groups than in sham controls or either of the withdrawal groups. There were significantly more labeled osteons per unit area in ovariectomized compared to sham controls, and rhPTH(1-34) did not increase these significantly above ovariectomized control values.

Intracortical porosity was greater in ovariectomized compared to shams, but not different between ovariectomized controls and PTH1. PTH5 and PTH5-W increased porosity above that seen in ovariectomized controls. Data from the rabbit studies suggested the hypothesis that the increase in porosity, accompanied by increased cortical bone, may be a structural response to maintain the biomechanical properties of rhPTH(1-34)-treated bone. There were no differences between ovariectomized and the other groups in formation period, osteoid width, wall width, or osteoid maturation at 18 months.

In summary, there were no differences in turnover rates between ovariectomized controls and either dose of rhPTH(1-34). Sham controls had a lower turnover rate than either ovariectomized controls or rhPTH(1-34)-treated animals. When rhPTH(1-34) was withdrawn for 6 months, turnover rates decreased significantly, but BMD and biomechanical strength measures remained higher than ovariectomized controls. Normal values for osteoid width and maturation time intracortically for all groups indicates treatment did not cause any defect in the normal timing of the mineralizing process. Normal values for wall width indicate that treatment did not alter the normal balance between resorption and formation at the level of the individual BMU.

**Table 6 Histomorphometric Variables for Cortical Bone Measurements of the Humerus**

| Variable^{a} | Units | Description |
|---|---|---|
| Ac.F | cycles/year | Activation frequency |
| BFR/BS.Ec | µm/day | Bone formation rare, endocortical surface referent |
| BFR/BS.Ps | µm/day | Bone formation rate, periosteal surface referent |
| BFR/BV | %/year | Bone formation rate, bone volume referent |
| FP | days | Formation period |
| L.On.N/Ct.A | #/mm² | Number of fluorochrome labeled osteons per unit cortical area |
| MAR | µm/day | Mineral apposition rate, intracortical |
| MAR.Ec | µm/day | Mineral apposition rate, endocortical surface |
| MAR.Ps | µm/day | Mineral opposition rate, periosteal surface |
| MS/BS.Ee | % | Minealizing endocortical surface normalized to total endocortical surface |
| MS/BS.Ps | % | Mineralizing periosteal surface normalized to total periosteal surface |
| O.Wi | µm | Osteoid width |
| Rs.N/Ct.A | #/mm² | Number of resorption spates per unit cortical area |
| W.Wi | µm | Osteonal wall width |
| omt | days | Osteoid maturation time |
| Po | % | Porosity, the percentage of bone area occupied by spaces |
| B.Ar | mm² | Bone area, the total area within the periosteal surface |
| Ct. Ar | mm² | Cortical area, the area of bone within the periosteal surface (includes porosities) |
| Mc.Ar | mm² | Medullary cavity area |

| | | |
|---|---|---|
| ^{a}Nomenclature is that recommended in Journal of Bone and Mineral Research, 1987. | | |

**Table 7 Cortical Histomorphometry of the Humerus Midshaft of Ovariectomized Primates at 18 Months (n=121)**

| Variable^{b} | Sham | OVX | PTH1 | TH1-W | PTH5 | PTH5-W |
|---|---|---|---|---|---|---|
| Ac.F | 1.85 ± 1.87^{a,c} | 6.06 ± 3.31 | 7.69 ± 4.96 | 3.05 ± 2.15^{a} | 8.70 ± 3.97 | 2.05 ± 1.46^{a} |
| BFR/BS.Ec | 7.08 ± 3.80 | 20.93 ± 19.23 | 18.14 ± 13.95 | 14.89 ± 10.32 | 34.04 ± 19.01 | 12.73 ± 16.33^{a} |
| BFR/BS.Ps | 3.79 ± 3.07 | 9.12 ± 7.58 | 8.53 ± 10.54 | 3.60 ± 3.84^{a} | 8.99 ± 5.81 | 5.79 ± 4.05 |
| BFR/BV | 2.13 ± 2.06^{a} | 9.16 ± 5.37 | 19.23 ± 5.93 | 4.39 ± 3.48^{a} | 12.93 ± 5.94^{a} | 2.21 ± 1.73^{a} |
| FP | 82.73 ± 41.06 | 65.94 ± 19.79 | 63.44 ± 10.02 | 64.94 ± 11.99 | 81.97 ± 95.63 | 88.63 ± 52.90 |
| L.On.N/Ct.A | 0.28 ± 0.27^{a} | 1.03 ± 0.52 | 1.26 ± 0.71 | 0.50 ± 0.36^{a} | 1.45 ± 0.47^{a} | 0.38 ± 0.26^{a} |
| MAR | 0.91 ± 0.33^{a} | 1.07 ± 0.19 | 0.98 ± 0.12^{a} | 1.06 ± 0.27 | 1.03 ± 0.23 | 0.85 ± 0.28^{a} |
| MAR.Ec | 0.48 ± 0.19^{a} | 0.75 ± 0.25 | 0.66 ± 0.15 | 0.66 ± 0.16 | 0.75 ± 0.14 | 0.63 ± 0.17 |
| MAR.Ps | 0.62 ± 0.24 | 0.69 ± 0.23 | 0.89 ± 0.95 | 0.54 ± 0.15^{a} | 0.66 ± 0.17 | 0.82 ± 0.15 |
| MS/BS.Ec | 3.09 ± 6.49^{a} | 20.99 ± 18.04 | 25.19 ± 17.14 | 11.74 ± 14.41^{a} | 40.47 ± 24.68^{a} | 8.93 ± 15.39^{a} |
| MS/BS.Ps | 1.81 ± 3.59^{a} | 10.03 ± 10.49 | 8.59 ± 5.73 | 3.86 ± 4.83^{a} | 11.00 ± 9.63 | 2.30 ±3.76^{a} |
| O.Wi | 3.77 ± 0.92 | 4.04 ± 0.91 | 3.66 ± 0.67 | 3.96 ± 0.83 | 3.94 ± 1.13 | 3.76 ± 0.83 |
| Rs.N/Ct.A | 0.12 ± 0.17^{a} | 0.21 ± 0.13 | 0.28 ± 0.18 | 0.12 ± 0.07^{a} | 0.43 ± 0.26^{a} | 10.19 ± 0.18 |
| W.Wi | 63.23 ± 13.61 | 68.63 ± 15.09 | 61.36 ± 7.79 | 63.12 ± 17.35 | 65.29 ± 9.43 | 63.82 ± 8.31 |
| omt | 4.58 ± 1.26^{a} | 3.87 ± 1.04 | 3.76 ± 0.70 | 3.86 ± 0.74 | 6.45 ± 13.85 | 5.07 ± 2.65 |
| Po | 1.32 ± 0.60^{a} | 2.61 ± 1.40 | 4.65 ± 4.78 | 2.23 ± 1.60 | 6.78 ± 4.23^{a} | 6.40 ± 4.22^{a} |
| B.Ar | 53.12 ± 5.50 | 52.71 ± 7.09 | 54.22 ± 5.97 | 54.94 ± 6.55 | 55.81 ± 6.24 | 58.16 ± 8.79^{a} |
| Ct.Ar | 37.40 ± 3.75 | 35.35 ± 5.04 | 37.61 ± 3.86 | 38.10 ± 4.83 | 40.96 ± 4.30^{a} | 40.83 ± 5.80^{a} |
| Me.Ar | 15.72 ± 4.07 | 17.47 ± 4.22 | 16.61 ± 3.77 | 16.84 ± 3.74 | 14.85 ± 4.72^{a} | 17.34 ± 6.05 |

| | | | | | | |
|---|---|---|---|---|---|---|
| Abbreviations: OVX=ovariectomized; PTH1=rhPTH(1-34) 1 µg/kg for 18 months; PTH1-W= withdrawal after treatment with rhPTH(1-34) 1 µg/kg for 12 months; PTH5=rhPTH(1-34) 5 µg/kg for 18 months; PTH5-W=withdrawal after treatment with rhPTH(1-34) 5 µg/kg for 12 months. ^{a}Statistically significant compared to OVX controls (p<0.05). ^{b}See table 4.2 for description of variables. ^{c}Data are expressed as mean ± standard error of the mean (SEM). | | | | | | |

The analysis by histomorphometry and polarized Fourier transform infrared microscopy revealed that administration of PTH improved bone quality by replacing old bone (large crystallites) with young bone (range of sized crystallites, tending to smaller size). Further, upon withdrawal of PTH from monkeys given low doses, there is an additional benefit as the matrix becomes optimally mineralized, and the crystallites mature. The data derived from histomorphometry and Fourier transform infrared microscopy show an unexpected benefit on bone quality of the cortical bone as optimal mineralization occurs the mineral phase matures.

### 3D Finite Element Modeling Studies

Examination of the middle 500 µm slice of L-5 showed a 21% increase in BMD for PTH compared to ovariectomized that was due to a 27% increase in BMC with no change in the cross sectional area. Analysis of the centrum from PTH showed a 73% increase in BV/TV that was due to a 30% increase in Tb.Th and 37% greater Tb.N, compared to ovariectomized.. Connectivity analysis for this region showed a 140% greater node density (node/tissue volume) and 286% greater node-to-node struts for the PTH vertebra.

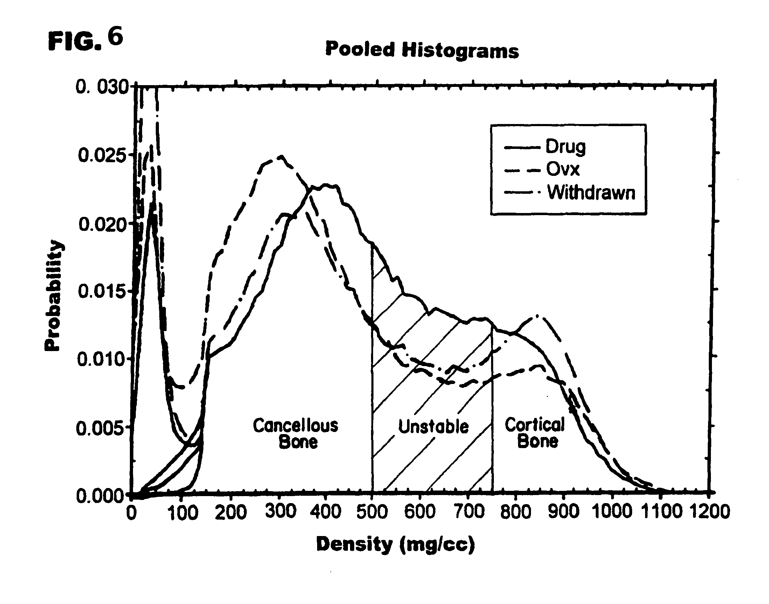

Histogram distribution analysis of bone voxel densities for PTH showed a decrease in the proportion of low densities (0-355 mg/cc), an increase in middle densities (356-880 mg/cc), with little effect on the high density voxels (887-1200 mg/cc), compared to ovariectomized (Fig. 6). Most striking was the shift to a greater bone voxel density in the cortical bone compartment following withdrawal of treatment for 6 months (Fig. 6).

The proportion of vertebral bone elements (voxels) that fell within a certain range of BMD values was calculated. The BMD ranges chosen were the following: low BMD, 0-300 mg/cc; medium BMD, 300-700 mg/cc; high BMD, 700-1000 mg/cc; and cortical BMD, >1000 mg/cc (Table 8). Compared to ovarectomized controls, PTH treatment significantly decreased the volume of low BMD bone and increased the volume of medium BMD bone. After withdrawal of PTH, there was a decrease in medium BMD bone and an increase in high BMD bone, indicating that medium BMD bone became more dense.

**Table 8. Percentages of L5 vertebral volume grouped by BMD values (mean±SEM)**

| Treatment | low BMD (%) | medium BMD(%) | high BMD (%) | cortical BMD (%) |
|---|---|---|---|---|
| ovariectomized | 30.4±2.2 | 48.7±1.6 | 19.9±1.2 | 0.9±0.3 |
| PTH | 17.7±1.6* | 58.9±1.9* | 22.8±2.7 | 0.6±0.3 |
| PTH-W | 22.4±1.3* | 49.7±1.2 | 27.0±1.6* | 0.8±0.2 |

**Table 9. BMC at the midlevel of the L5 vertebra and vertebral effective strain**

| Treatment | BMC (mg) | effective strain (ustrain) |
|---|---|---|
| ovariectomized | 37.2±1.6 | 701±64 |
| PTH | 47.4±1.5* | 447±36* |
| PTH-W | 44.2±1.2* | 539±34* |

| | | |
|---|---|---|
| *statistically different (p<0.05 by Fisher's PLSD test) | | |

The data summarized in Figure 6 show that L-5 vertebra from cynomolgus monkeys treated for 18 months with PTH respond with significant increases in bone mass, trabecular thickness, and trabecular connectivity, with marginal effects on the outer dimensions (X-Area) of the vertebra. Analysis of the distribution of bone elements in L-5 showed that the heavily mineralized bone regions change the least with no evidence of bone sclerosis. Rather, it is the porous trabecular bone that responded the most to PTH. The shift in BMD led to a substantial reduction in the axial strain, indicating mechanical improvement. As clearly shown in the histograms of PTH and ovariectomy BMD, PTH converted the low density bone voxels into medium density voxels with no significant effect on the high density voxels.

The average mechanical strain in the vertebra was reduced 36% by PTH treatment and remained 23% below OVX after withdrawal of PTH. This study indicates that withdrawal of PTH treatment for 6 months did not lead to resorption of newly formed bone, but instead there was a beneficial redistribution of medium density bone into lower and higher density bone. This redistribution led to continued strain reduction in the vertebrae and thus improved mechanical function.

### Discussion

This primate study indicates that PTH, given in the absence of other medicines that might affect bone, benefits both cortical and trabecular bone to increase overall skeletal bone mass. Moreover, withdrawal of PTH did not result in significant loss of benefits associated with PTH treatment over a period of at least 2 remodeling cycles.

Surrogate markers have been used in other trials to indicate activity in bone, and they assumed that changes in value reflect changes in bone mass. Although there are published data on humans and primates to show that both formation and resorption markers increase, consistent with activation of bone turnover, for example, during early menopause or in active disease states, the high turnover is considered to be indicative of bone loss. In adolescence, high turnover during maturation of the human skeleton has been less well studied, but is accompanied by an anabolic gain in bone mass. Such a phenomenon would be totally unexpected in drug therapy of osteoporosis, according to current art. Thus, the increase in bone turnover markers is inconsistent with the known anabolic effect of PTH to increase bone mass and strength, as shown by data in the present study.

The data from this 18 month study on cynomolgus monkeys supports the following unexpected findings:
- Overall significant increase in total skeletal mass
- Significant increase in bone mass and strength at the femur neck.
- No evidence of "steal" from cortical bone to increase trabecular bone. Increase in bone mass and strength were statistically significant at sites enriched for either cortical bone (femur neck) or trabecular bone (lumbar vertebrae). At purely cortical bone sites (femur mid-diaphysis), there was a trend for PTH to stabilize or slightly increase bone mass and strength, compared to ovariectomized controls.
- Changes in bone markers in ovariectomized monkeys (and humans) do not reflect the beneficial, anabolic effects of PTH on the skeleton. Use of body fluids from primates in the present study allows development of new and more valid surrogate markers.
- Retention of the gain in bone mass and strength for at least 2 remodeling cycles after withdrawal of treatment.

This PTH primate study differs from published studies on rhesus and cynomolgus monkeys in that it used a large sample size to provide appropriate statistical power to detect differences that may not have been apparent in previous much smaller studies; controls included both ovariectomized primates (used in published studies) and sham operated, but intact primates. The latter control group has not been previously reported in this type of study, so that some of the benefits of PTH, and the restoration of certain measures to sham control levels, compared to values for ovariectomized animals were assessed for the first time.

### Conclusions

This 18-month study in mature, feral, ovariectomized (OVX) cynomolgus monkeys, *Macaca fascicularis,* assured efficacy and safety in bone following treatment with rhPTH(1-34) for either 12 months followed by withdrawal of treatment for 6 months, or treatment for 18 months. rhPTH(1-34) significantly increased bone mass and strength of the spine and femur neck above ovariectomized controls to levels equivalent to or greater than those of sham controls. In ovariectomized monkeys treated with rhPTH(1-34), measures of calcium homeostatis (serum calcium, phosphate, and 1,25-dihydroxyvitamin D) were restored to that of sham controls. Serum, urine, and histomorphometry measures used to assess bone turnover showed rhPTH(1-34) maintained formation rates equivalent to or higher than those of ovariectomized controls, while biochemical markers of bone resorption remained equivalent to those of shame controls. In all animals treated with rhPTH(1-34) for up to 18 months, pharmacokinetic measures did not change with time, and there was no accumulation of rhPTH(1-34). There was no evidence of sustained hypercalcemia or kidney pathology after 18 months of treatment. There were no changes in mineralization or remodeling periods. The net gain in skeletal bone mineral content observed with rhPTH(1-34) may be explained by increased bone formation rate and bone forming surface with little or no effect on bone resorption. There were significant increases in bone mineral content, bone mineral density and biomechanical measures of strength, including toughness and stiffness, at clinically relevant sites such as the spine, femur neck and proximal tibia.

rhPTH(1-34) increased the rate of turnover in cortical bone of midshaft of the humerus and radius, but did not significantly alter bone mass or biomechanical measures of strength compared to either ovariectomized or sham controls. However, the increase in cortical width and/or cortical bone area is consistent with an increase in cross-sectional moment of inertia, a measure of strength and stiffness. rhPTH(1-34) had no significant effects on the intrinsic material properties of cortical bone. Endocortical bone formation was stimulated, thus increasing cortical width and intracortical porosity. It appears that these changes in porosity are responsible for maintaining the elasticity of the bone.

In monkeys, 12 months of treatment with rhPTH(1-34) followed by withdrawal for 6 months was associated with smaller, but still significant, gains in bone mass and strength in the spine and femur neck. Following withdrawal, no significant effects were noted on the cortical bone midshaft of the humerus and radius. Bone markers and histomorphometry showed trends to return to the low turnover values measured in sham controls.

In vivo mechanistic studies in rodents showed that genes associated with anabolic outcomes of rhPTH(1-34) are upregulated within 1 to 6 hours, and the increase in bone forming surfaces can be detected within 24 hours after the first dose in the absence of detectable effects on resorption. rhPTH(1-34) appears to recruit osteoprogenitors in S-phase, and stimulate their differentiation into osteoblasts, thereby rapidly increasing the percent bone forming surfaces. Single or multiple injections of rhPTH(1-34) may be given within a 1-hour period to induce the anabolic effect in bone. However, when the equivalent dose is given in young rats as multiple injections over 6 hours or 8 hours, the anabolic effect was abrogated, suggesting that brief, limited exposure to rhPTH(1-34) is required to induce the anabolic effect.

In summary, rhPTH(1-34) is anabolic on the bone in monkeys and rabbits, increasing bone mass and biomechanical strength measures at clinically relevant sites such as the lumbar spine and femur neck by selective stimulation of bone formation. The increases in bone turnover, endocortical surface formation, and porosity, detected by histomorphometry at cortical sites, did not alter bone mass or biomechanical measures of bone strength, but did increase the cross sectional moment of inertia by increasing cortical bone area and/or width.

These studies demonstrate that administration of parathyroid hormone receptor activators, such as recombinant human PTH(1-34) improve bone quality both during and following treatment. In fact, administering PTH once daily for 18 months, or at the same doses for 12 months followed by a 6 month withdrawal phase, showed marked improvement of the quality of cortical bone of the humerus as analyzed by histomorphometry and polarized Fourier transform infrared (FTIR) microscopy. This analysis revealed that administration of PTH improved bone quality by replacing old bone (large crystallites) with young bone (range of sized crystallites, tending to smaller size). Thus, administration of PTH can increase cortical bone quality, improve mineralization, and accelerate mineralization and replacement of old bone by new bone.

Further, upon withdrawal of PTH from monkeys given low doses, there is an additional benefit as the matrix becomes more optimally mineralized, and the crystallites mature. That is, at low doses, PTH can have additional benefits during the withdrawal phase of treatment by enhancing mineralization. These data indicate benefits of a finite regime of treatment with PTH followed by a withdrawal period to achieve enhanced benefit. Current definitions of bone quality do not include these aspects of improved mineralization.

In earlier studies of a treatment phase of PTH followed by a phase of no treatment, the treatment phase was less than 1 month. The prolonged but finite treatment phase of 18-24 months followed by a period of at least 2 remodeling cycles has not previously been explored. The continued benefit in primates after withdrawal of treatment is in marked contrast to results achieved in rodents upon dosing with PTH. Studies of rats have uniformly shown that bone is rapidly lost following withdrawal of treatment. Gunness-Hey, M. and Hock, J. M. (1989) Bone 10: 447-452; Shen, V. et al. (1993) J. Clin Invest 91: 2479-2487; Shen, V. et al. (1992) Calcif. Tissue Int. 50: 214-220; and Mosekilde, L. et al. (1997) Bone 20: 429-437.

Such a method of enhancing bone mineralization has not previously been observed and is unexpected, revealing a new method by which PTH strengthens and toughens bone and can prevent fractures. This new method includes enhancing and regulating mineralization, to provide tougher, stiffer, more fracture resistant bone. Such beneficial effects require more than new matrix formation. These findings indicate that PTH has benefits in patients with immobilized bones or skeletons, or in skeletons deficient in mineral, provided there is also adequate calcium and vitamin D supplementation.

**Example 2- Increased Bone Strength and Density, and Reduced Fractures Upon Administration of rhPTH(1-34) to Humans**

| | |
|---|---|
| Number of Subjects: | rhPTH(1-34): 1093 enrolled, 848 finished. |
| | Placebo: 544 enrolled, 447 finished. |
| Diagnosis and Inclusion Criteria: | Women ages 30 to 85 years, postmenopausal for a minimum of 5 years, with a minimum of one moderate or two mild atraumatic vertebral fractures. (for reference) |
| Dosage and Administration: | Test Product (blinded) |
| | rhPTH(1-34): 20 µg/day, given subcutaneously |
| | rhPTH(1-34): 40 µg/day, given subcutaneously |
| | Reference Therapy (blinded) |
| | Placebo study material for injection |
| Duration of Treatment: | rhPTH(1-34): 17-23 months (excluding 6-month run-in phase) |
| | Placebo: 17-23 months (excluding 6-month run-in phase) |
| Criteria for Evaluation: | Spine x-ray; serum biological markers (calcium, bone-specific alkaline phosphatase, procollagen I carboxy-terminal propeptide); urine markers (calcium, N-telopeptide, free deoxypyridinoline); 1,25-dihydroxyvitamin D; bone mineral density: spine, hip, wrist, and total body; height; population pharmacokinetics; bone biopsy (selected study sites). |

### Patient Characteristics

| | Placebo (N=544) | PTH-20 (N=541) | PTH-40 (N=552) | p-value |
|---|---|---|---|---|
| Caucasian | 98.9% | 98.9% | 98.4% | 0.672 |
| Age | 69.0±7.0 | 69.5±7.1 | 69.9±6.8 | 0.099 |
| Years post menopausal | 20.9±8.5 | 21.5±8.7 | 21.8±8.2 | 0.273 |
| Hysterectomized | 23.8% | 23.1% | 21.6% | 0.682 |
| Uterus + 0 or 1 ovary | 57 | 51 | 58 | |
| Uterus + 2 ovaries | 61 | 57 | 51 | |
| Unknown | 11 | 17 | 10 | |
| Previous osteoporosis drug use | 14.9% | 15.5% | 13.0% | 0.479 |
| Baseline spine BMD | 0.82±0.17 | 0.82±0.17 | 0.82±0.17 | >0.990 |
| Baseline # of vert. fx | | | | >0.990 |
| 0 | 54 (10.4%) | 45 (8.8%) | 54 (10.1%) | |
| 1 | 144(27.8%) | 159(31.1%) | 169 (31.6%) | |
| 2 | 128 (24.7%) | 128(25.0%) | 125(23.4%) | |
| 3 | 75 (14.5%) | 67 (13.1%) | 81 (15.1%) | |
| 4 | 59 (11.4%) | 49 (9.6%) | 45 (8.4%) | |
| 5 | 28 (5.4%) | 31 (6.1 %) | 21 (3.9%) | |
| 6 | 13 (2.5%) | 20 (3.9%) | 25 (4.7%) | |
| 7 | 6 (1.2%) | 7 (1.4%) | 10 (1.9%) | |
| 8 | 9 (1.7%) | 5 (1.0%) | 3 (0.6%) | |
| 9 | 1 (0.2%) | 0 | 2 (0.4%) | |
| 10 | 1 (0.2%) | 1 (0.2%) | 0 | |
| Unspecified | 26 | 29 | 17 | |

### Results

Data from this clinical trial including a total of 1637 women treated with recombinant human parathyroid hormone (1-34), rhPTH(1-34) 0, 20, or 40 µg/kg/day, and supplemented with vitamin D and calcium, for 18-24 months, showed results reported in Tables 15-19.

Table 10 illustrates data showing the reduction upon treatment with PTH of the number and severity of vertebral fractures. Comparing all PTH treated patients with placebo, the overall reduction in number of patients with vertebral fractures was 67% (p<0.001), with a 65% reduction (p<0.001) at 20 µg/day PTH compared to placebo, and a 69% reduction at 40 µg/day PTH compared to placebo (Table 10). Comparing all PTH treated patients with placebo, the overall reduction in number of patients with multiple vertebral fractures was 81% (p<0.001), with a 77% reduction (p<0.001) at 20 µg/day PTH compared to placebo, and a 86% reduction at 40 µg/day PTH compared to placebo. Comparing all PTH treated patients with placebo, the overall reduction in number of patients with moderate to severe vertebral fractures was 84% (p<0.001), with a 90% reduction (p<0.001) at 20 µg/day PTH compared to placebo, and a 78% reduction at 40 µg/day PTH compared to placebo (Table 10).

**Table 10. Effect of treatment with PTH on number and severity of vertebral fractures.**

| | Placebo | 20 µg/day PTH | 40 µg/day PTH |
|---|---|---|---|
| | (n*=448) | (n=444) | (n=434) |
| Number and percentage of patients with new vertebral fractures | 64 | 22 | 19 |
| | (14.3%) | (5.0%) | (4.4%) |
| Number and percentage of patients with 2 or more new vertebral fractures | 22 | 5 | 3 |
| | (4.9%) | (1.1%) | (0.7%) |
| Number and percentage of patients with new moderate to severe fractures** | 42 | 4 | 9 |
| | (9.4%) | (0.9%) | (2.1%) |

| | | | |
|---|---|---|---|
| *n = number of patients with both baseline and endpoint x-rays ** Moderate fracture results in more than 25% loss of vertebral height (or an equivalent measure). Severe fracture results in more than 40% loss of vertebral height (or an equivalent measure). Fractures are as defined by Genant et al. (1993) Vertebral fracture assessment using a semiquantitative technique; J. Bone & Min Res 81137-1148. | | | |

Table 11 illustrates the effect of treatment with PTH on the number of fractures at various non-vertebral bones throughout the body. The number of fractures apparently decreased at each of the hip, radius, ankle, humerus, ribs, foot, pelvis, and other sites (Table 11). The reduction is statistically significant when viewed as the reduction in the total number of fractures among the PTH treated patients compared to the placebo treated patients. The reduction is even more significant when considered as the reduction in the total number of fractures of hip, radius, ankle, humerus, ribs, foot, and pelvis among the PTH treated patients compared to the placebo treated patients (Table 11).

**Table 11: Effect of treatment with PTH on number of non-vertebral fractures.**

| | | | | p-values | | | |
|---|---|---|---|---|---|---|---|
| | Placebo (N=544) | PTH-20 (N=541) | PTH-40 (N=552) | Overall | PTH-pbo* | 20-pbo | 40-pbo |
| Hip | 4 | 2 | 3 | 0.718 | 0.474 | 0.417 | 0.690 |
| Radius | 13 | 7 | 10 | 0.404 | 0.236 | 0.180 | 0.504 |
| Ankle | 4 | 2 | 2 | 0.601 | 0.313 | 0.417 | 0.403 |
| **Humerus** | 5 | 4 | 3 | 0.767 | 0.534 | 0.744 | 0.465 |
| **Ribs** | 10 | 5 | 5 | 0.277 | 0.109 | 0.197 | 0.184 |
| **Foot** | 4 | 1 | 4 | 0.374 | 0.474 | 0.181 | 0.983 |
| **Pelvis** | 3 | 1 | 0 | 0.171 | 0.076 | 0.319 | 0.081 |
| **Other** | 16 | 14 | 9 | 0.338 | 0.296 | 0.723 | 0.146 |
| **Total** | 53 | 34 | 32 | 0.024 | 0.007 | 0.036 | 0.015 |
| **Total w/o "Other"** | 41 | 21 | 24 | 0.013 | 0.003 | 0.010 | 0.025 |

| | | | | | | | |
|---|---|---|---|---|---|---|---|
| *Placebo (pbo) | | | | | | | |

The effect of PTH on bone mineral content (BMC), bone mineral density (BMD), and bone area were determined by dual energy absorptiometry (DEXA), and the results are reported in Tables 12-14. PTH administration caused apparent increases in BMC at the patient's lumbar spine, femur and hip, wrist, and throughout the patient's whole body (Table 12). Treatment with PTH caused significant increases in the patient's BMD at the lumbar spine, femur and hip (Table 13). The increases at the lumbar spine, femur and hip were statistically significant with p<0.001 (Table 13). Bone area apparently increased upon PTH treatment for the patient's lumbar spine, femur and hip (Table 14). The increases were statistically significant for the lumbar spine and hip neck (Table 14).

The effect of PTH on the whole body the measure of bone quantity and quality, BMC, is particularly significant. This whole body effect indicates that the amount of bone in the patient's body is increasing. PTH does not merely result in moving bone mass from one portion of the patient's body to another. Instead, treatment with PTH increases the amount and quality of bone in the patient's body.

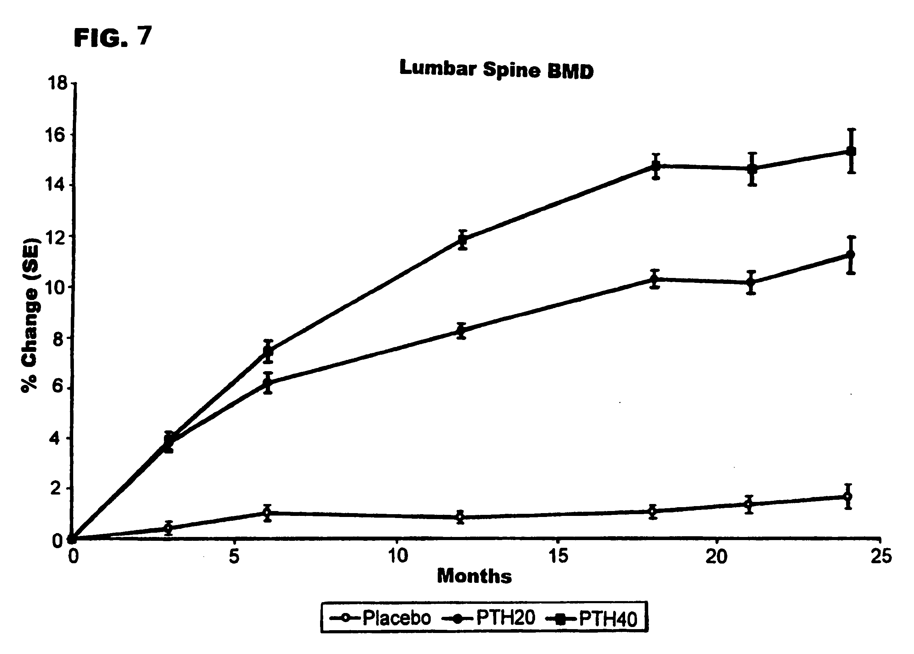

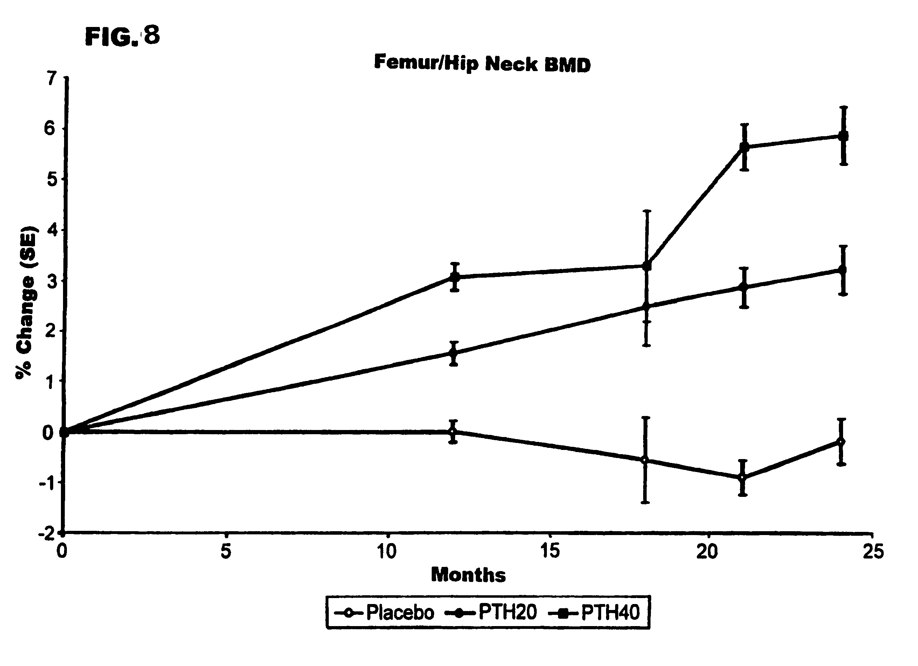

Figures 7 and 8 illustrate the increases over time in lumbar spine BMD and femur/hip neck BMD, respectively, for PTH treated and placebo control patients. The patient's lumbar spine BMD increases steadily for at least about 18 months, with no or a less significant increase over the subsequent months. The patient's femur/hip BMD apparently increases for at least 18 months, and may increase upon further duration of PTH treatment.

**Table 12. Effect of PTH on bone mineral content expressed as endpoint % change (SD) from baseline**

| | Placebo | PTH-20 | PTH-40 | p-value |
|---|---|---|---|---|
| Lumbar spine | 1.60 (6.92) | 11.85 (8.83) | 16.62 (11.1) | <0.001 |
| Femur/Hip | | | | |
| Total | -0.38 (5.18) | 3.50 (6.26) | 4.78 (6.70) | <0.001 |
| Neck | -0.51 (7.06) | 2.99 (7.26) | 5.80 (8.71) | <0.001 |
| Trochanter | 0.98 (14.97) | 5.68 (15.58) | 6.53 (15.33) | <0.001 |
| Intertrochanter | -0.23 (6.28) | 3.59 (7.32) | 4.99 (7.79) | <0.001 |
| Ward's triangle | 0.01 (14.75) | 5.36 (14.78) | 8.86 (17.02) | <0.001 |

| Wrist | | | | |
|---|---|---|---|---|
| Ultradistal | -1.67 (7.44) | -0.25 (6.53) | -1.88 (7.97) | 0.184 |
| 1/3 radius | -1.19 (6.12) | -1.37 (4.51) | -3.04 (6.09) | 0.025 |
| Whole body | -0.74 (4.76) | 1.30 (4.48) | 2.28 (5.44) | <0.001 |

**Table 13. Effect of PTH on bone mineral density expressed as endpoint % change (SD) from baseline**

| | Placebo | PTH-20 | PTH-40 | p-value |
|---|---|---|---|---|
| Lumbar spine | 1.13 (5.47) | 9.70 (7.41) | 13.7 (9.69) | <0.001 |
| Femur/Hip | | | | |
| Total | -1.01 (4.25) | 2.58 (4.88) | 3.60 (5.42) | <0.001 |
| Neck | -0.69 (5.39) | 2.79 (5.72) | 5.06 (6.73) | <0.001 |
| Trochanter | -0.21 (6.30) | 3.50 (6.81) | 4.40 (7.45) | <0.001 |
| Intertrochanter | -1.29 (4.53) | 2.62 (5.52) | 3.98 (5.96) | <0.001 |
| Ward's triangle | -0.80 (11.73) | 4.19 (11.93) | 7.85 (13.24) | <0.001 |

| Wrist | | | | |
|---|---|---|---|---|
| Ultradistal | -1.89 (7.98) | -0.05 (7.14) | -1.76 (7.20) | 0.108 |
| 1/3 radius | -1.22 (3.37) | -1.94 (4.07) | -3.17 (4.62) | 0.001 |

**Table 14. Effect of PTH on bone area expressed as endpoint % change (SD) from baseline**

| | Placebo | PTH-20 | PTH-40 | p-value |
|---|---|---|---|---|
| Lumbar spine | 0.46 (2.97) | 2.52 (3.52) | 3.34 (3.72) | <0.001 |
| Femur/Hip | | | | |
| Total | 0.54 (3.02) | 0.84 (3.16) | 1.05 (2.98) | 0.144 |
| Neck | 0.04 (4.60) | 0.27 (4.91) | 0.81 (5.56) | 0.035 |
| Trochanter | 0.95 (12.75) | 1.99 (12.16) | 1.92 (11.30) | 0.197 |
| Intertrochanter | 1.01 (5.17) | 1.01 (4.99) | 1.01 (4.89) | 0.964 |
| Ward's triangle | 0.44 (7.60) | 1.13 (7.34) | 0.99 (8.06) | 0.309 |

| Wrist | | | | |
|---|---|---|---|---|
| Ultradistal | 0.25 (6.40) | -0.25 (6.00) | -0.39 (4.80) | 0.653 |
| 1/3 radius | -0.02 (5.73) | 0.52 (3.40) | 0.01 (4.42) | 0.586 |

In summary, the data presented above indicate that patients treated with PTH have reduced fractures. Specifically, PTH treatment reduced by more than 66% the number of patients with prior vertebral fractures who suffered new vertebral fractures. PTH treatment also reduced by more than 78% the number of patients with prior vertebral fractures who suffered new, multiple vertebral fractures. In addition, PTH decreased the severity of vertebral fractures, with a significant reduction by 78% in the number of patients with moderate or severe fractures. Patients receiving PTH benefited from a significant reduction in all non-vertebral fractures (including fractures of hip, radius, wrist, pelvis, foot, humerus, ribs or ankle) with significance at a level of p<0.007. Bone quality increases as well. Patients with prior fracture benefited from a significant increase in bone mineral content of the hip, spine and total body. This increase indicates that fracture reduction at these sites can occur as early as after 12 months of therapy.

### Discussion

These data on fractures are the first data on fracture reduction by PTH in humans. These findings demonstrate an improvement in bone quality and bone strength, like the preclinical data reported hereinabove. These results also show benefits in bone quality and strength at non-vertebral sites. The findings of a reduction in the numbers of fractures sustained during the 18-23 month period of treatment has not previously been observed in clinical or preclinical studies.

The question of whether PTH alone increases toughness and strength of bone to improve resistance to fracture has not previously been tested in humans. The published literature has consistently suggested that PTH must be given in combination with an anti-resorptive or estrogen. Previous, published clinical trials included patient populations too small to determine a significant reduction of fracture. In one study the benefits of PTH alone could not be assessed because there were no placebo controls. In a second study, employing the commonly accepted definition of fracture, no reduction in fracture was observed.

The findings of a reduction of fractures at combined non-vertebral sites is particularly unexpected in light of the common belief that PTH has negative effects at such sites. Common dogma holds that PTH will increase cortical porosity and therefore weaken bone, especially early in therapy. Further, this dogma asserts that cortical bone sites are at high risk of fracture and that PTH will offer no benefit in fracture reduction at non-vertebral sites. The dogma also holds that PTH alone is unlikely to be efficacious and will require concurrent anti-resorptive therapy to block negative effects on cortical bone. The present data demonstrate the previously unobserved benefits of PTH given to patients receiving vitamin D and calcium supplements. Unexpectedly, PTH strengthens bone to reduce the number of new fractures in a patient at risk for multiple fractures of the spine, at risk for additional non-vertebral fractures, at risk for moderate to severe additional fractures of the spine, and the like.

This clinical study on post-menopausal women showed particular benefits from treating patients with low dose (20 µg/day) since the dose of PTH (which, at high doses, could show side effects in some patients) was reduced, but fracture prevention and fracture reduction was retained, and similar to those noted at the high dose (40 µg/day). The FT-IR monkey data provide a possible, but not limiting, mechanistic explanation. The monkey study shows that low dose PTH increased crystal formation and accelerated mineralization in cortical bone. In addition, low dose monkeys showed additional benefits after withdrawal, as PTH enhanced mineral content of the bone. The present data demonstrate the novel finding that PTH given at low doses to patients receiving vitamin D and calcium supplements, is effective in preventing both vertebral and non-vertebral fractures. Contrary to popular belief, PTH strengthens bone at non-vertebral sites to prevent new fractures or reduce the severity of fractures, apparently by improving the mineralization and mineral content of the bone.

The invention has been described with reference to various specific and preferred embodiments and techniques. However, it should be understood that many variations and modifications may be made while remaining within the scope of the invention. All publications and patent applications in this specification are indicative of the level of ordinary skill in the art to which this invention pertains.

## Claims

1. Human PTH(1-34) for use in preventing or reducing the incidence or severity of vertebral and/or non-vertebral fracture in a male human at risk for or having osteoporosis, wherein the PTH(1-34) is administered by subcutaneous injection at a dose of 10 to 40 µg/day.

2. Human PTH (1-34) for use according to claim 1, wherein the male human has osteoporosis and the PTH (1-34) is administered once daily for 1-7 days for a period ranging from 3 months for up to 3 years.

3. Human PTH (1-34) for use according to any preceding claim, wherein the male human also receives effective doses of calcium and vitamin D.

4. Human PTH(1-34) for use according to any preceding claim, wherein the PTH(1-34) is administered for 12-24 months and is then withdrawn for at least 6 months.

5. Human PTH(1-34) for use according to any preceding claim, wherein the PTH(1-34) is administered as a stabilized solution including a stabilizing agent, a buffering agent and a preservative.

## Patentansprüche

1. Human-PTH(1-34) zur Verwendung bei der Vorbeugung und Reduzierung des Auftretens oder der Schwere von Wirbelfrakturen und/oder extravertebralen Frakturen bei einem Mann, bei dem ein Osteoporoserisiko besteht bzw. der an Osteoporose leidet, wobei das PTH (1-34) durch subkutane Injektion in einer Dosis von 10 bis 40 µg/Tag verabreicht wird.

2. Human-PTH(1-34) zur Verwendung nach Anspruch 1, wobei der Mann an Osteoporose leidet und das PTH(1-39) einmal pro Tag 1-7 Tage lang über einen Zeitraum von 3 Monaten bis zu 3 Jahren verabreicht wird.

3. Human-PTH(1-34) zur Verwendung nach einem der vorhergehenden Ansprüche, wobei dem Mann auch Calcium und Vitamin D in wirksamen Dosen verabreicht wird.

4. Human-PTH(1-34) zur Verwendung nach einem der vorhergehenden Ansprüche, wobei das PTH(1-34) 12-24 Monate lang verabreicht und dann mindestens 6 Monate lang abgesetzt wird.

5. Human-PTH(1-34) zur Verwendung nach einem der vorhergehenden Ansprüche, wobei das PTH(1-34) als stabilisierte Lösung, die ein Stabilisierungsmittel, einen Puffer und ein Konservierungsmittel beinhaltet, verabreicht wird.

## Revendications

1. PTH(1-34) humaine destinée à être utilisée pour la prévention ou la réduction de la fréquence ou de la sévérité d'une fracture vertébrale et/ou non vertébrale chez un mâle humain à risque ou atteint d'ostéoporose, **caractérisée en ce que** la PTH(1-34) est administrée par injection sous-cutanée à une dose de 10 à 40 µg/jour.

2. PTH(1-34) humaine destinée à être utilisée selon la revendication 1, **caractérisée en ce que** le mâle humain est atteint d'ostéoporose et la PTH(1-34) est administrée une fois par jour pendant 1-7 jours pendant une période allant de 3 mois jusqu'à 3 ans.

3. PTH(1-34) humaine destinée à être utilisée selon l'une ou l'autre des revendications précédentes, **caractérisée en ce que** le mâle humain reçoit également des doses efficaces de calcium et de vitamine D.

4. PTH(1-34) humaine destinée à être utilisée selon l'une quelconque des revendications précédentes, **caractérisée en ce que** la PTH(1-34) est administrée pendant 12-24 mois et est ensuite arrêtée pendant au moins 6 mois.

5. PTH(1-34) humaine destinée à être utilisée selon l'une quelconque des revendications précédentes, **caractérisée en ce que** la PTH(1-34) est administrée en solution stabilisée comportant un agent stabilisant, un agent tampon et un agent conservateur.
